(19) Europäisches Patentamt
European Patent Office
Office européen des brevets

(11) Publication number: **0 409 345 A2**

## EUROPEAN PATENT APPLICATION

(21) Application number: 90201935.5

(22) Date of filing: 17.07.90

(51) Int. Cl.⁵: **C12Q 1/34**

The title of the invention has been amended (Guidelines for Examination in the EPO, A-III, 7.3).

(30) Priority: 21.07.89 US 384190

(43) Date of publication of application:
**23.01.91 Bulletin 91/04**

(84) Designated Contracting States:
**AT BE CH DE ES FR GB GR IT LI LU NL SE**

(71) Applicant: **EASTMAN KODAK COMPANY**
**343 State Street**
**Rochester, New York 14650(US)**

(72) Inventor: **Detwiler, Richard Linn, c/o**
**EASTMAN KODAK COMPANY**
**Patent Department, 343 State Street**
**Rochester, New York 14650(US)**
Inventor: **Daggett, Stephen Greg., c/o**
**EASTMAN KODAK COMPANY**
**Patent Department, 343 State Street**
**Rochester, New York 14650(US)**
Inventor: **Arter, Thomas Charles, c/o**
**EASTMAN KODAK CO.,**
**Patent Department, 343 State Street**
**Rochester, New York 14650(US)**
Inventor: **Fricker, Robert Frank, c/o EASTMAN**
**KODAK CO.,**
**Patent Department, 343 State Street**
**Rochester, New York 14650(US)**
Inventor: **Ferris, Robert James, c/o EASTMAN**
**KODAK CO.,**
**Patent Department, 343 State Street**
**Rochester, New York 14650(US)**

(74) Representative: **Nunney, Ronald Frederick**
**Adolphe et al**
**Kodak Limited Patent Department Headstone**
**Drive**
**Harrow Middlesex HA1 4TY(GB)**

(54) Analytical element and method for the determination of creatinine.

(57) A multilayer analytical element comprises a support having thereon in the following order:
   a) a first reagent layer comprising sarcosine oxidase, a rate limiting amount of creatinine amidohydrolase; a peroxidative substance and imidazole leuco dye which is capable of providing a detectable dye in the presence of said peroxidative substance and hydrogen peroxide;
   b) a second reagent layer containing creatinine amidinohydrolase; and
   c) a porous spreading layer;
   characterized in that a high NEG oxidase activity sarcosine oxidase having an activity of at least 300 I.U./m² relative to N-ethyl glycine is present in i) the porous spreading layer; ii) a separate layer between the spreading layer and the layer containing creatine amidinohydrolase; iii) the layer containing creatine amidinohydrolase or iv) all of such layers i) - iii).

NEG INTERFERENCE

(graph with y-axis labeled "BIAS v ZERO NEG (mg/L CREATININE)" and x-axis labeled "NEG OXIDASE ACTIVITY SACROSINE I.U./M$^2$")

O — HIGH NEG OXIDASE ACTIVITY SACROSINE OXIDASE IN SPREAD LAYER.
Δ — " " " " " " " SEPARATE GELATIN LAYER.
⊙ — ELEMENT EPA 88304343.2

FIG. 1

## DRY CREATININE ANALYTICAL ELEMENT AND METHOD FOR ITS USE

The present invention relates to clinical chemistry. In particular, it relates to an analytical element and method for the determination of creatinine in aqueous liquids, such as biological fluids.

The determination of the intermediate and end products of protein metabolism is important in clinical chemistry, and particularly in the diagnosis of kidney function. The products of this metabolism include creatinine and creatine.

Enzymatic assays for creatinine have been developed using enzymes specific for creatinine and creatine, respectively, and the following reaction sequence:

(1) creatinine + water $\rightleftharpoons$ creatine

(2) creatine + water $\rightarrow$ urea + sarcosine The first reaction is catalyzed by creatinine amidohydrolase whereas the second one is catalyzed by creatine amidinohydrolase.

U.S. Patent 4,182,399 discloses an analytical element for the determination of creatinine or creatine or both. This element exhibits a large clinically unacceptable bias in creatinine assay samples from patients on lidocaine therapy. It is believed that a metabolite of lidocaine, N-ethyl glycine (NEG), causes this bias.

This bias problem is somewhat ameliorated with an analytical element described in EP-A-0 291 321. That element comprised an absorbent carrier material containing 200-1,200 I.U./m$^2$ of the enzyme creatinine amidohydrolase, 35,000-65,000 I.U./m$^2$ of the enzyme creatine amidinohydrolase and 2,000-3,500 I.U./m$^2$ of a bacillus species of sarcosine oxidase and a leuco dye which is capable of providing a detectable dye in the presence of hydrogen peroxide and a peroxidative substance.

The creatine amidinohydrolase is present in a manner such that it is substantially inert to the leuco dye. The creatinine amidohydrolase is present in a rate limiting amount. All three of the enzymes are present at a pH of 6.5 to 6.9.

The problem is that the latter element still exhibits bias in creatinine assay samples from patients being treated with lidocaine.

The present invention provides an improved element for the assay of creatinine. The bias in such assays caused by the lidocaine metabolite NEG is significantly reduced compared to the elements of U.S. Patent 4,182,399 and EP-A-0 291 321.

The element comprises a multilayer analytical element for the determination of creatinine comprising a support having thereon in the following order:

a) a first reagent layer comprising sarcosine oxidase, a rate limiting amount of creatinine amidohydrolase; a peroxidative substance and imidazole leuco dye which is capable of providing a detectable dye in the presence of said peroxidative substance and hydrogen peroxide;

b) a second reagent layer containing creatine amidinohydrolase; and

c) a porous spreading layer; characterized in that a high NEG oxidase activity sarcosine oxidase having an activity of at least 300, preferably 600, I.U./m$^2$ relative to N-ethyl glycine is present in i) the porous spreading layer; ii) a separate layer between 'the spreading layer and the layer containing creatine amidinohydrolase; iii) the layer containing creatine amidinohydrolase or iv) all of such layers i) - iii).

The invention also provides a method for the determination of either creatinine or creatine comprising the steps of:

A. in the presence of a peroxidative substance, contacting a sample of a liquid suspected of containing either creatinine or creatine with a multilayer analytical element according to the present invention; and

B. determining the detectable dye formed as a result of the presence of either creatinine or creatine.

The improved analytical element of this invention uses an approach to minimize or eliminate NEG caused assay bias that is entirely different from that disclosed in EP-A-0 291 321. An essential feature of the present element is the use of sarcosine oxidase having a high NEG oxidase activity. The use of the latter sarcosine oxidase is made possible by the discovery that sarcosine oxidases from different sources have different NEG oxidase activities relative to NEG.

Sarcosine oxidase is, by definition, an enzyme which utilizes sarcosine as a substrate. However, it has been found that sarcosine oxidase from most if not all sources can also use N-ethyl glycine (NEG) as a substrate, although NEG is not as good a substrate as sarcosine. The activity of an enzyme is generally defined as the number of micromoles of a substrate converted per minute. The activity of sarcosine oxidase is determined by using sarcosine as a substrate. If the same test for activity is performed using NEG as a substrate, the resulting activity can be termed "NEG oxidase" activity. This term will be used in the description of this invention. It should be understood, however, that the "NEG oxidase" may be a fundamental property of sarcosine oxidase, as opposed to being a separate enzyme, present as an impurity.

3

FIG. 1 is a graph comparing the effect of NEG bias on elements of this invention compared to the elements of EP-A-0 291 321.

FIG. 2 is another graph of the effect of NEG bias on an element of the invention compared to several elements of EP-A-0 291 321.

The present invention relates to the determination (qualitative or quantitative measurement) of creatinine in aqueous liquids. In particular, the invention can be used to assay biological fluids of animals and humans. Such fluids include, but are not limited to, whole blood, plasma, sera, lymph, bile, urine, spinal fluid, sputum, perspiration and the like as well as stool secretions. It is also possible to assay fluid preparations of human or animal tissue such as skeletal muscle, heart, kidney, lungs, brains, bone marrow, skin and the like. Preferably, human serum or urine is assayed with this invention.

The multilayer elements of this invention can have three or more discrete zones, either in the same layer or in superimposed layers, at least one of which is preferably a porous spreading zone. The other zones can be reagent zones or registration zones as those zones are known in the art, additional spreading zones, radiation-blocking or filter zones, subbing zones, barrier zones, etc. The zones are generally in fluid contact with each other, meaning that generally fluids, reagents and reaction products (e.g. color dyes) can pass or be transported between superposed regions of adjacent zones. Preferably, the zones are separately coated in superposed layers.

The porous spreading zone can be self-supporting (i.e. composed of a material rigid enough to maintain its integrity), but preferably it is carried on a separate nonporous support. Such a support can be any suitable dimensionally stable, and preferably, nonporous and transparent (i.e. radiation transmissive) material which transmits electromagnetic radiation of a wavelength between 200 and 900 nm. A support of choice for a particular element should be compatible with the intended mode of detection (transmission or reflectance spectroscopy). Useful supports can be prepared from polystyrene, polyesters [e.g. poly-(ethylene terephthalate)], polycarbonates, cellulose esters (e.g. cellulose acetate), etc.

The porous spreading zone can be prepared from any suitable fibrous or non-fibrous material or mixtures of either or both as described in U. S. Patents 4,292,272; 3,992,158; 4,258,001 and 4,430,436 and Japanese Patent Publication 57(1982)-101760. It is desirable that the spreading zone be isotropically porous, meaning that the porosity is the same in each direction in the zone as caused by interconnected spaces or pores between particles, fibers, polymeric strands, etc.

The assay of this invention is accomplished with the following sequence of reactions (1)-(4):

(1) creatinine + water $\rightleftharpoons$ creatine
(2) creatine + water $\rightarrow$ urea + sarcosine
(3) sarcosine + oxygen + water $\rightarrow$ glycine + formaldehyde + hydrogen peroxide
(4) hydrogen peroxide + leuco dye $\rightarrow$ detectable dye.

These reactions are catalyzed by creatinine amidohydrolase, creatine amidinohydrolase, sarcosine oxidase and a peroxidative substance, respectively.

Previous efforts to minimize the interference of lidocaine metabolites on creatinine and creatine assay have been directed toward preventing the following reaction (5) from going forward:

(5) N-ethyl glycine + $O_2$ $\rightarrow$ $H_2O_2$

Sarcosine oxidase catalyzes this reaction. The $H_2O_2$ formed in this reaction combines with the $H_2O_2$ formed in reaction (3), supra , to form a detectable dye, thereby preventing accurate creatinine and creatine assays.

In the present invention the interference is essentially eliminated by promoting completion of reaction (5) through use of a sarcosine oxidase that has a higher NEG oxidase activity relative to N-ethyl glycine than sarcosine oxidases used heretofore in analytical elements for assaying creatinine.

In the multilayer elements of the invention, the conversion of NEG to $H_2O_2$ is carried to completion in the spreading layer; in a separate layer between the spreading layer and the layer comprising creatine amidinohydrolase or in the layer comprising creatine amidinohydrolase. The reaction is completed by the first read point. The first read point is at about 4 minutes. The $H_2O_2$ produced in reaction (5) causes little or no assay interference because it is essentially complete before the first read point.

The present invention provides a relatively simple, automated means to rapidly and economically measure either creatinine or creatine or both with the same analytical element. Separate analytical compositions or elements are therefore not needed for the different analytes. The assay of this invention is rapid, allowing measurement of either analyte within, for example, about 5 minutes. It is also possible to avoid tedious blanking steps with the present invention. The complex equipment used for solution assays described in Japanese Patent Publication 58(1983)-009699 is therefore avoided with the present invention.

These advantages are possible with the present invention because it is a kinetic assay for creatinine performed with the analytical element described herein. This element contains creatinine amidohydrolase and creatine amidinohydrolase which promote the two enzymatic reactions (1) and (2) noted supra .

However, the creatinine amidohydrolase is present in a rate limiting amount so that once endogenous creatine has completely reacted, the rate of dye formation is due totally to the presence of creatinine. Background is minimized by putting the reagents into the element in such a manner that they do not interfere with each other. In particular, the creatine amidinohydrolase is present in a manner such that it is substantially inert to the leuco dye.

Creatinine amidohydrolase and creatine amidinohydrolase can be obtained commercially from a number of sources. Several species of each enzyme, isolated from various microbial sources, are described in U.S. Patents 3,806,416 and 4,039,384. Any of the species can be used in the practice of this invention. The creatinine amidohydrolase and the creatine amidinohydrolase, both obtained from a strain of Flavobacterium and described in U.S. Patent 4,039,384, are useful.

Sarcosine oxidase can also be obtained commercially from a number of sources. Sarcosine oxidase from Bacillus species is useful in the layer containing creatinine amidohydrolase. The NEG oxidase activity of this sarcosine oxidase is less than 300 I.U./m$^2$ relative to NEG.

The high NEG oxidase activity sarcosine oxidase having an activity of at least 300 I.U./m$^2$ relative NEG from any source will be useful. A preferred high NEG oxidase activity sarcosine oxidase is an Arthobacter species.

Peroxidative substances useful in this invention include peroxidase. A peroxidase is an enzyme which will catalyze a reaction wherein hydrogen peroxide oxidizes another substance. The peroxidases are generally conjugated proteins containing iron porphyrin. Peroxidase occurs in horseradish, potatoes, fig tree sap and turnips, milk and white blood cells. It also occurs in micro-organisms and can be produced by fermentation. Certain synthetic peroxidases are also known. Peroxidase is a preferred peroxidative substance, but other substances which are not enzymes are also useful. Many of these are commercially available.

The leuco dyes useful in this invention are imidazole leuco dyes which are generally colorless in the leuco form, but which can be oxidized to a detectable colored dye in the presence of hydrogen peroxide and a peroxidative substance. Useful leuco dyes include di- and triarylimidazoles such as those described in U.S. Patent 4,089,747; EP-A-0 122 641 and Jap. Patent Publication 58(1983)-045, 557. Particularly useful imidazole leuco dyes are the triarylimidazoles described in U.S. Patent 4,089,747, including, e.g. 2-(3,5-dimethoxy-4-hydroxyphenyl)-4,5-bis(4-dimethyl-aminophenyl)imidazole, 2-(4-hydroxy-3-methoxyphenyl )-4,5-bi( p dimethylaminophenyl)-1H-imidazole, 2-(3-ethoxy-4-hydroxyphenyl-4,5-bis( p dimethylaminophenyl)-1H-imidazole, 2-(4-hydroxy-3,5-dimethoxyphenyl)-4-[4-(dimethylamino)-phenyl]-5-(2-furyl)imidazole, 2-(4-hydroxy-3,5-dimethoxyphenyl)-4,5-di(2-furyl)imidazole, 2-(3,5-dimethoxy-4-hydroxyphenyl)-4-[4-(dimethylamino)phenyl]-5-phenethylimidazole and 2-(3,5-dimethoxy-4-hydroxyphenyl)-4-[4-(dimethylamino)-phenyl]-5-benzylimidazole.

The elements of this invention can also contain one or more other addenda commonly included in the elements for various manufacturing or operational advantages. Such addenda include surfactants, ion chelating agents, buffers, solvents, hardeners, antioxidants, coupler solvents, and the like.

The creatinine amidohydrolase is present in the element in a rate limiting amount. This means that the amount of this enzyme in relation to the amount of creatine amidinohydrolase is such that the forward direction of reaction (1) noted above is rate controlling. The specific amount of creatinine amidohydrolase can be readily determined by a skilled clinical chemist. Generally, the amount is preferably from 200 to 1,200, I.U./m$^2$. Creatine amidinohydrolase can be present in any amount as long as it is not a rate limiting amount. In other words, reaction (2), noted above, is not to be controlling in the assay. The specific amount of this enzyme can be readily determined by a skilled clinical chemist.

As used in the context of this disclosure and the claims, I.U. represents the International Unit for enzyme activity defined as one I.U. being the amount of enzyme activity required to catalyze the conversion of 1 micromole of substrate per minute under standard pH and temperature conditions for the enzyme. For the preferred enzyme preparations used in this invention, these standard conditions are 30° C and pH 8.0 for creatinine amidohydrolase, 37° C and pH 7.4 for creatine amidinohydrolase, 37° C and pH 7.4 for sarcosine oxidase and 25° C and pH 7.0 for peroxidase.

The other reagents useful in the assay are present in suitable amounts readily determined by a skilled clinical chemist. Representative amounts are illustrated in the examples below.

It is critical in the practice of this invention that the creatine amidinohydrolase and leuco dye are present in a manner such that the enzyme is substantially inert to the dye. This means that the two reagents are incorporated in the element in such a manner that the enzyme does not adversely affect the leuco dye. This can be accomplished in a number of ways. For example, the enzyme can be used in a highly pure form so that the leuco dye is not affected by any impurities. More practically, however, the enzyme cannot be obtained in a highly pure form. In such cases, either or both the enzyme and dye can be encapsulated or otherwise isolated from each other in the element until the assay is carried out. Preferably, the enzyme and leuco dye are located in different zones or layers of the element so that they do not mix until the time of the assay.

A variety of different elements, depending on the method of assay, can be prepared in accordance with the present invention. Elements can be configured in a variety of forms, including elongated tapes of any desired width, sheets, slides or chips.

The assay of this invention can be manual or automated. In general, in using the dry elements, creatinine or creatine determination is made by taking the element from a supply roll, chip packet or other source and physically contacting it with a sample (e.g. 1 to 200 $\mu$l) of the liquid to be tested so that the sample and reagents within the element become mixed. Such contact can be accomplished in any suitable manner, e.g. dipping or immersing the element into the sample or, preferably, by spotting the element by hand or machine with a drop of the sample with a suitable dispensing means.

After sample application, the element is exposed to any conditioning, such as incubation, heating or the like, that may be desirable to quicken or otherwise facilitate obtaining any test result.

The rate of dye formation is then measured with suitable reflection or transmission spectro-photometric equipment and procedures. Generally, for creatine determination, a dye measurement is made prior to substantial conversion of creatinine, i.e. soon after sample-element contact, e.g. prior to about 1 minute after sample-element contact. This measurement determines endogenous creatine because the rate limiting amount of creatinine amidohydrolase in the element has converted substantially no creatinine to creatine at this point.

For creatinine determination, at least two dye measurements are made after substantially all endogenous creatine and NEG has been converted enzymatically to reaction products. Generally, the first measurement is made about 4 minutes after sample-element contact. Another measurement is made thereafter in order to determine the rate of dye formation, and hence, the amount of creatinine. This sequence of measurements allows the use of the present element to measure either or both creatinine and creatine.

As stated hereinbefore, a metabolite of lidocaine, N-ethyl glycine (NEG), sometimes creates clinical bias when the analytical elements of the aforementioned U.S. Patent and U.S. Patent Application are used to assay creatinine. The belief that N-ethyl glycine is the interferent is supported by the similarities in bias observed in patient samples containing known amounts of N-ethyl glycine. This belief is also supported by independent studies published in Clinical Chemistry , Vol. 34, No. 12 (1988) pp. 2559-2572 and No. 10, pp. 2144-2148.

A typical element according to the present invention can have the following format and configuration.

| | | |
|---|---|---|
| Spreading Layer | Titanium dioxide | 20–80 g/m$^2$ |
| | Cellulose acetate | 2–10 g/m$^2$ |
| | BRIJ 78 surfactant | 0.3–1.5 g/m$^2$ |
| | TRITON X–405 surfactant | 0.5–5 g/m$^2$ |
| | ESTANE resin | 1–5 g/m$^2$ |
| | High NEG Oxidase Activity Sarcosine Oxidase (This sarcosine oxidase may be in the spreading layer; the gel layer; the reagent layer or all three). | 300–3000 I.U./m$^2$ NEG Oxidase Activity |
| Subbing Layer | Poly(N–isopropylacryl-amide) | 0.1–1 g/m$^2$ |

| | | |
|---|---|---|
| Gel Layer | Gelatin (hardened) | $1-20 \ g/m^2$ |
| | High NEG Oxidase Activity Sarcosine Oxidase (This sarcosine oxidase may be the spreading layer; the gel layer; the reagent layer or all three.) | $300-3000 \ I.U./m^2$ NEG Oxidase Activity |

| | | |
|---|---|---|
| | Gelatin | $1-20 \ g/m^2$ |
| | N-tris-(hydroxymethyl)-methyl-2-aminomethane sulfonic acid buffer | $0.5-5 \ g/m^2$ |
| Reagent Layer | (Ethylenedinitrilo)tetra-acetic acid | $0.1-1 \ g/m^2$ |
| | Creatine amidinohydrolase | $5,000-50,000 \ I.U./m^2$ |
| | Ascorbic acid oxidase | $1,000-10,000 \ I.U./m^2$ |
| | TRITON X-100 surfactant | $0.1-2.5 \ g/m^2$ |
| | High NEG Activity Oxidase Sarcosine Oxidase (This sarcosine oxidase may be in the spreading layer; the gel layer; the reagent layer or all three.) | $300-3000 \ I.U./m^2$ NEG Oxidase Activity |

| | | |
|---|---|---|
| | Gelatin (hardened) | $1-20 \ g/m^2$ |
| | Sarcosine oxidase | $500-10,000 \ I.U./m^2$ |
| | Peroxidase | $500-80,000 \ I.U./m^2$ |
| | 5,5-dimethyl-1,3-cyclo-hexanedione | $0.01-1 \ g/m^2$ |
| | 2,4-di-n-pentyl phenol | $0.5-5 \ g/m^2$ |

Creatinine amidohydrolase

$50-2,500$ I.U./m$^2$

| Registration/Reagent Layer | N-tris-(hydroxymethyl)-methyl-2-aminomethane sulfonic acid buffer | $0.5-5$ g/m$^2$ |
| | (Ethylenedinitrilo)tetra-acetic acid | $0.1-1$ g/m$^2$ |
| | TRITON X-100 surfactant | $0.1-2.5$ g/m$^2$ |
| | ALKANOL XC surfactant | $0.1-2.5$ g/m$^2$ |
| | 2,4-di-n-pentyl phenol | $1-5$ g/m$^2$ |
| | 2-(3,5-dimethoxy-4-hydroxyphenoy)-4,5-bis(4-methylamino-phenyl)imidazole | $0.1-1$ g/m$^2$ |

Poly(ethylene terephthalate) Support

This element was used to determine creatinine in the following manner. First, a calibration curve was prepared for creatinine by applying 10 µl samples of the appropriate calibrator fluids to separate elements of the invention, incubating and measuring the resulting dye at 670 nm. Reflectance density ($D_R$) readings were made at 231 and 300 seconds after addition of each sample. A calibration curve for creatinine was obtained by subtracting the reading at 231 seconds from the respective reading taken at 300 seconds and dividing by 1.217 for each calibrator sample. This curve enables one to determine the kinetic rate of reaction for creatinine.

Samples containing unknown amounts of creatinine were similarly determined.

Example 1

An assay was carried out according to the above calibration procedure on NEG spiked and unspiked serum samples. The level of the spike was 18 mg of NEG/L.

This example shows that the interference caused by NEG is sharply reduced when the high NEG oxidase activity sarcosine oxidase is in either the spreading layer or a separate layer as previously specified compared to the analytical element of EP-A-0 291 321.

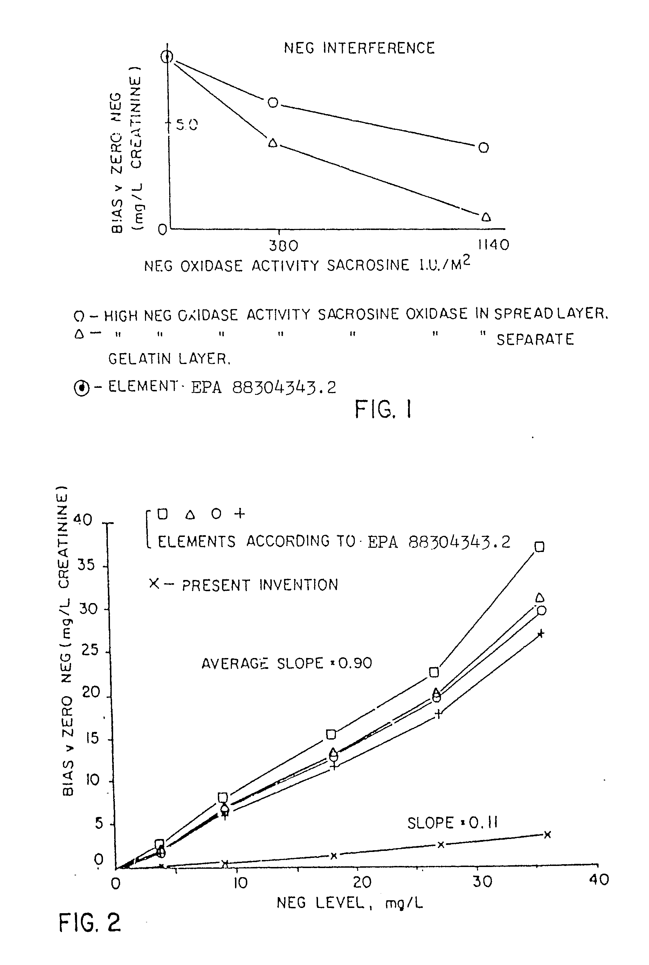

The results of this test are shown in the graph in Figure 1. This graph shows that a high NEG oxidase activity sarcosine oxidase coverage of 300 I.U./m$^2$ reduces NEG interference by 50% compared to the element of EP-A-0 291 321.

Example 2

Figure 2 is a graph summarizing a test on the analytical element of this invention in which the high NEG oxidase activity sarcosine oxidase relative to NEG is included in a gelatin layer located between the spreading layer and the reagent layer which contains creatine amidinohydrolase.

In this test a pool of serum samples from lidocaine-free patients was spiked with varying levels of NEG. The NEG spiked serum samples were then tested on the Ektachem analyzer using both the analytical element of EP-A-0 291 321 and the above embodiment of the element of this invention. The tests were conducted as described above for preparation of the calibration curve. The resulting biases are shown in Figure 2.

The results show a dramatic reduction (about 85%) in the amount of interference or bias from NEG in assay results using elements of the invention compared to the element of EP-A-0 291 321.

**Claims**

1. A multilayer analytical element comprising a support having thereon in the following order:
   a) a first reagent layer comprising sarcosine oxidase, a rate limiting amount of creatinine amidohydrolase; a peroxidative substance and imidazole leuco dye which is capable of providing a detectable dye in the presence of said peroxidative substance and hydrogen peroxide;
   b) a second reagent layer containing creatine amidinohydrolase; and
   c) a porous spreading layer;
   characterized in that a high NEG oxidase activity sarcosine oxidase having an activity of at least 300 I.U./m$^2$ relative to N-ethyl glycine is present in i) the porous spreading layer; ii) a separate layer between the spreading layer and the layer containing creatine amidinohydrolase; iii) the layer containing creatine amidinohydrolase or iv) all of layers i) - iii).

2. The element of claim 1 wherein the high NEG oxidase sarcosine oxidase is in a layer between the spreading layer and the layer containing the creatine amidinohydrolase.

3. The element of claim 1 wherein the high NEG oxidase sarcosine oxidase is in the spreading layer.

4. The element of claim 1 wherein the high NEG oxidase sarcosine oxidase is in the layer containing the creatine amidinohydrolase.

5. The elements of claims 2, 3 and 4 wherein the coverage of the high NEG oxidase activity sarcosine oxidase has an activity of at least 600 I.U./m$^2$.

6. A method for the determination of creatinine comprising the steps of:
   A) contacting a sample of a liquid human serum suspected of containing creatinine or creatine with a multi layer analytical element according to any one of the preceding claims for the determination of either creatinine or creatine, and
   B) determining the detectable dye formed as a result of the presence of either creatinine or creatine.

FIG. 1

FIG. 2